# EUROPEAN PATENT SPECIFICATION

(11) **EP 3 201 607 B1**
(45) Date of publication and mention of the grant of the patent: **30.12.2020**
(21) Application number: 15846111.1
(22) Date of filing: 28.09.2015
(51) Int. Cl.: G01N 21/64, A61B 6/00, A61B 1/04, A61B 5/00

(54) **IMAGING A TARGET FLUOROPHORE IN A BIOLOGICAL MATERIAL IN THE PRESENCE OF AUTOFLUORESCENCE**
BILDGEBUNG EINES ZIELFLUOROPHORS IN EINEM BIOLOGISCHEN MATERIAL IN ANWESENHEIT VON AUTOFLUORESZENZ
IMAGERIE D'UN FLUOROPHORE CIBLE DANS UNE MATIÈRE BIOLOGIQUE EN PRÉSENCE D'AUTO-FLUORESCENCE

(30) Priority: 29.09.2014 US 201462056830 P
(43) Date of publication of application: 09.08.2017
(73) Proprietor: Novadaq Technologies ULC, Burnaby, BC V5A 4W2 (CA)
(72) Inventor: MORIYAMA, Eduardo Hiroyuki, Richmond, British Columbia V6Y 4E4 (CA); SZE, Chun Ho, Coquitlam, British Columbia V3C 5G6 (CA)
(74) Representative: V.O.
(86) International application number: PCT/CA2015/050973
(87) International publication number: WO 2016/049756

(56) References cited:
- FR-A1- 2 944 104
- JP-A- 2009 291 554
- US-A- 5 827 190
- US-A1- 2010 016 669
- US-A1- 2011 063 427
- US-A1- 2012 165 627
- US-A1- 2013 203 082
- US-A1- 2013 286 176
- US-A1- 2013 342 674
- BAUMGARTNER R ET AL: "A FLUORESCENCE IMAGING DEVICE FOR ENDOSCOPIC DETECTION OF EARLY STAGE CANCER. ÖINSTRUMENTAL AND EXPERIMENTAL STUDIES", 1 January 1990 (1990-01-01), SELECTED PAPERS ON OPTICAL FIBERS IN MEDICINE; [SPIE MILESTONE SERIES], BELLINGHAM, SPIE, US, PAGE(S) 513 - 517, XP000467844, * figures 1-3 * * *

## Description

### CROSS-REFERENCE TO RELATED APPLICATION

This application is related to U.S. Provisional Application No. 62/056830, filed on September 29, 2014.

### TECHNICAL FIELD

The present invention relates generally to fluorescence imaging, and in particular to imaging a target fluorophore in a biological material in the presence of autofluorescence.

### BACKGROUND

In the life sciences, fluorescence is typically used as a non-invasive method of identifying and analyzing biological materials. Specific targets in the biological material such as for example, proteins, nucleic acids, lipids, cells and cell components, stem cells or small molecules can be labeled with an extrinsic or exogenous fluorophore, and thus subsequently imaged. Biological materials also naturally fluoresce, which is known as intrinsic fluorescence or "autofluorescence" because it occurs in the absence of exogenously administered fluorophores. Autofluorescence is believed to originate from various endogenous fluorophores in biological materials, including for example nicotinamide adenine dinucleotide (NADH), elastin, collagen, flavins, amino acids and porphyrins.

Autofluorescence and fluorescence emission can be generated and recorded as images when light with the appropriate excitation wavelengths illuminates the biological material. However, autofluorescence, which is the result of a combination of fluorophores and is characterized by broad emission spectra extending over several hundred nanometers, can interfere with the ability to detect the emission of a specific fluorophore, when the emission spectra of the fluorophore and the autofluorescence overlap. In such instances, in addition to reducing signal detection sensitivity by masking the fluorescence of the fluorophore of interest, autofluorescence may also decrease the specificity of detection by providing false positive results.

One approach to addressing this problem is to utilize means to reduce or minimize the detected emission signal that is contributed by autofluorescence of the biological material. The prior art describes methods to reduce autofluorescence by employing various pre-treatments of the biological material prior to image acquisition. However, such techniques may also degrade the quality of the biological material itself, and are typically not suitable for *in vivo* applications. Alternatively, if the autofluorescence emission itself cannot be mitigated, it is possible to minimize the contribution of signal from autofluorescence to image data by means of digital manipulation of any acquired fluorescence images. For example, in images containing the combined signal from both the fluorophore of interest and autofluorescence, some of these methods rely on acquiring estimates of the "pure" autofluorescence signal and using such estimates to remove autofluorescence by a weighted subtraction. Other methods use statistical correlation techniques to correct for the additive autofluorescence signal. These image data manipulation techniques are described in prior art references and are generally limited by poor accuracy, by the need for small (i.e., low resolution) data sets, or by the need for significant post-processing. It is consequently desirable to establish a high resolution image processing technique to quickly and accurately distinguish the fluorescence emitted by a fluorophore of interest in a biological material from the autofluorescence emission in that same biological material.

The article: "A FLUORESCENCE IMAGING DEVICE FOR ENDOSCOPIC DETECTION OF EARLY STAGE CANCER - INSTRUMENTAL AND EXPERIMENTAL STUDIES"; SELECTED PAPERS ON OPTICAL FIBERS IN MEDICINE; BELLINGHAM, SPIE, US; 1990-01-01; BAUMGARTNER R ET AL; VOLUME MS 11; PAGES 513 - 517 discloses the following. A method of obtaining the fluorescence emission of DHE over the full spectral bandwidth (600-700 nm) and with elimination of any undesired tissue autofluorescence in the final image of a sample of bladder tissue. The method involves excitation of the sample with both violet and blue light excitation.

### SUMMARY

In accordance with one aspect of the invention, there is provided a method for extracting an image of a target fluorophore in a biological material according to appended claim 1. In the method a waveband for the target fluorophore emission overlaps a waveband for autofluorescence emission in the biological material. The method includes illuminating the biological material with a first excitation light to induce a first fluorescence emission arising from both autofluorescence of the biological material and fluorescence of the target fluorophore and with a second excitation light to induce a second fluorescence emission arising from the autofluorescence of the biological material, acquiring a first fluorescence image from the first fluorescence emission and a second fluorescence image from the second fluorescence emission, and processing the first and second fluorescence images to extract a third fluorescence image representing the target fluorophore, wherein relative intensities of the first and second excitation lights are modulated prior to acquiring the first and second fluorescence images. The processing may for example involve subtracting the second fluorescence image from the first fluorescence image.

The modulation of the relative intensities includes identifying a wavelength region in the first and second fluorescence emissions, wherein the wavelength region is a region where emission arising from the fluorophore is present in the first fluorescence emission and absent in the second fluorescence emission, selecting a waveband outside the wavelength region, calculating at the selected waveband a ratio of relative intensities of the first and second fluorescence emissions, and adjusting the relative intensities of the first and second excitation lights to adjust the corresponding first fluorescence emission, second fluorescence emission or both until a suitable calculated ratio is achieved. According to an embodiment, the ratio of relative intensities of the first and second fluorescence emissions may be calculated by dividing an area-under-the curve value corresponding to the first fluorescence emission by an area-under-the curve value corresponding to the second fluorescence emission.

In accordance with another aspect of the invention, there is provided a system for extracting an image of a target fluorophore in a biological material according to appended claim 13. In the system a waveband for the target fluorophore emission overlaps a waveband for autofluorescence emission in the biological material. The system includes means for illuminating the biological material with a first excitation light to induce a first fluorescence emission arising from both autofluorescence of the biological material and fluorescence of the target fluorophore and with a second excitation light to induce a second fluorescence emission arising from the autofluorescence of the biological material, means for acquiring a first fluorescence image from the first fluorescence emission and a second fluorescence image from the second fluorescence emission, means for modulating relative intensities of the first and second excitation lights prior to acquisition of the first and second fluorescence images, and means for processing the first and second fluorescence images to extract a third fluorescence image representing the target fluorophore. According to an embodiment, the means for illuminating includes an illumination module, the means for acquiring includes a fluorescence emission acquisition module, and the means for processing includes a processor module.

In the embodiments where the target fluorophore is porphyrin, for example, the first excitation light has a wavelength of about 405 nm, the second excitation light has a wavelength of about 450 nm, the selected waveband is about 600 nm, and the calculated ratio is about 1.

### BRIEF DESCRIPTION OF THE DRAWINGS

In accompanying drawings which illustrate embodiments of the invention,
FIG. **1** schematically illustrates an exemplary method according to an embodiment;
FIG. **2** illustrates fluorescence spectra arising from autofluorescence and porphyrin in urine (FIG. **2A**), autofluorescence (FIG. **2B**) in urine, and the differential spectrum corresponding to porphyrin alone obtained in accordance with the various embodiments (FIG. **2C**);
FIG. **3** illustrates fluorescence spectra of freshly obtained urine (**3A**) and photobleached urine (**3B**) at 405 nm and 450 nm according to an embodiment;
FIG. **4** illustrates images corresponding to the fluorescence spectra in FIG **3** where the left column (A, C, E) relates to freshly collected urine, and the right column (B, D, F) relates to photobleached urine, the top row (A, B) relates to fluorescence images from excitation at about 405 nm, the middle row (C, D) relates to fluorescence images from excitation at about 450 nm, and the bottom row (E, F) illustrates the differential images corresponding to the target fluorophore (porphyrin) obtained according to an embodiment;
FIG. **5A** illustrates an example *in vivo* fluorescence image of the subject's forearm when excited with 405 nm light, displaying autofluorescence from the forearm and porphyrin fluorescence; FIG. **5B** illustrates a fluorescence image of the same region of the forearm as in

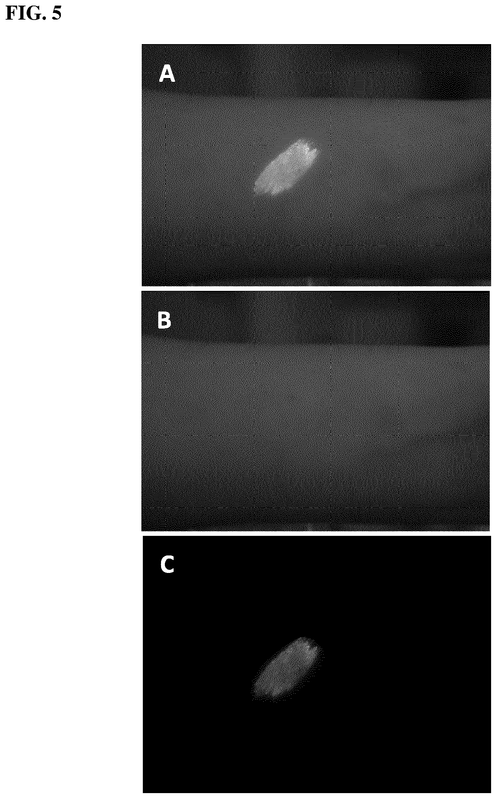

FIG. **5A** upon excitation with 450 nm light showing a reduction in autofluorescence to a level similar to the autofluorescence level in FIG **5A** where the porphyrin fluorescence is absent under the 450 nm excitation; FIG. **5C** illustrates a fluorescence image of porphyrin with the autofluorescence removed in accordance with an embodiment;

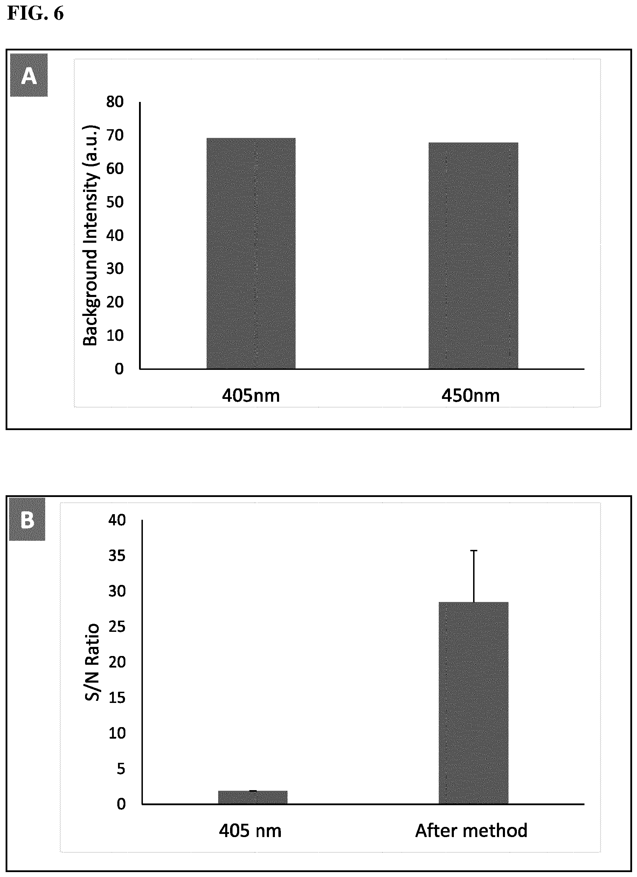

FIG. **6A** illustrates background intensity values at 405 nm and 450 nm after the excitation intensities were adjusted at 600 nm, and a lower than about 2% difference between background values was observed between excitations; FIG. **6B** illustrates a comparison of the signal-to-noise ratio from fluorescence images excited at 405 nm and after background was removed in accordance with an embodiment;

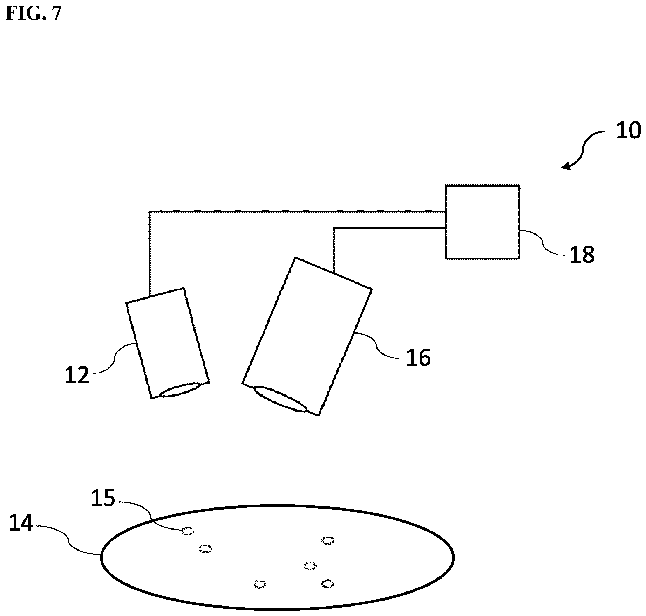

FIG. **7** illustrates a system for extracting an image of a target fluorophore in a biological material according to an embodiment;

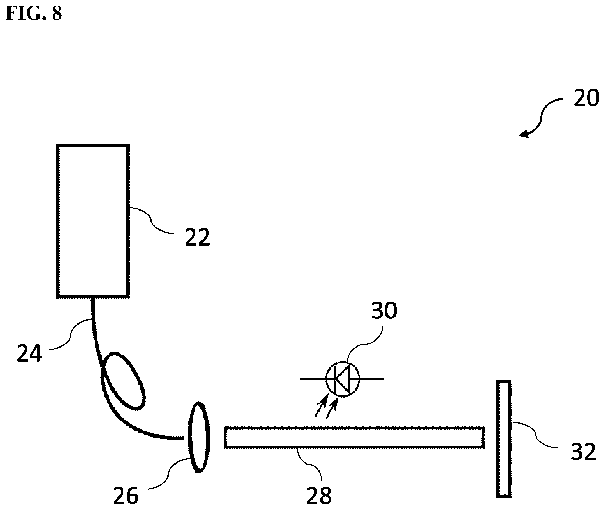

FIG. **8** illustrates an illumination module according to an embodiment; and

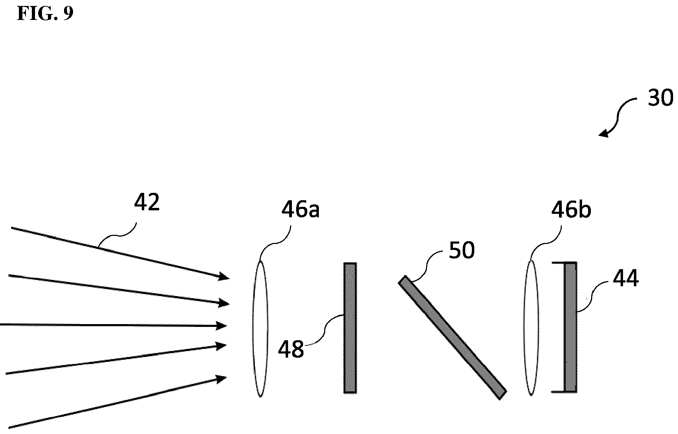

FIG. **9** illustrates a fluorescence emission acquisition module according to an embodiment.

### DETAILED DESCRIPTION

Reference will now be made in detail to implementations and embodiments of various aspects and variations of the invention, examples of which are illustrated in the accompanying drawings.

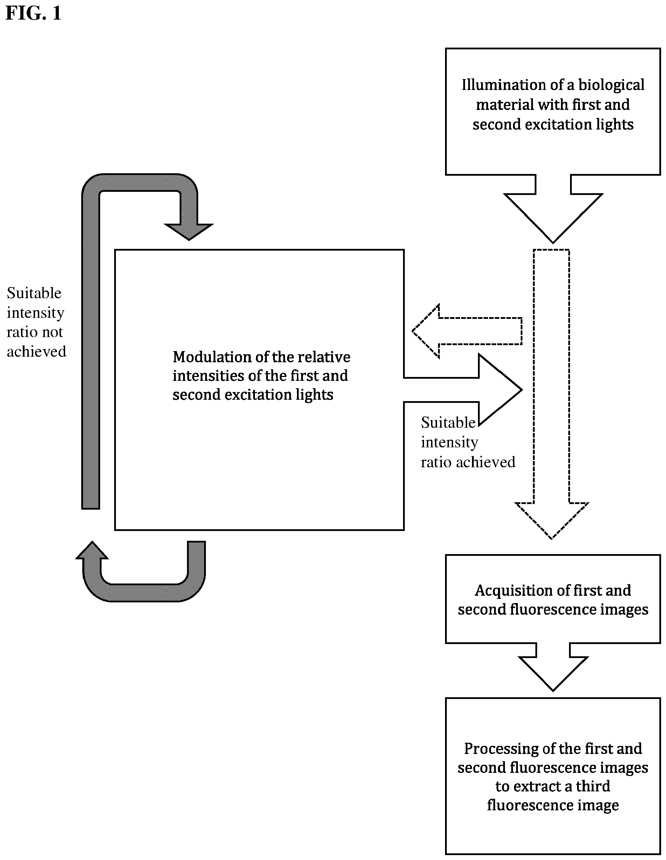

According to one aspect of the invention, there is provided a method for extracting an image of a target fluorophore in a biological material according to appended claim 1. In the method a waveband for the target fluorophore emission overlaps a waveband for autofluorescence emission in the biological material. Figure **1** schematically illustrates the method.

Referring to Figure 1, the method comprises illuminating the biological material with a first excitation light to induce a first fluorescence emission arising from both autofluorescence of the biological material and fluorescence of the target fluorophore and with a second excitation light to induce a second fluorescence emission arising from the autofluorescence of the biological material, acquiring a first fluorescence image from the first fluorescence emission and a second fluorescence image from the second fluorescence emission, and processing the first and second fluorescence images to obtain a third fluorescence image representing the target fluorophore, wherein relative intensities of the first and second excitation lights are modulated prior to acquiring the first and second fluorescence images.

In various embodiments, the biological material includes a material derived from, obtained from, or located in a biological subject (e.g., a mammal), and further includes a material *in vitro*, *in situ* or *in vivo.* Examples of the biological material include a biological tissue or fluid or a fraction thereof, an organ, a cell, a cell line, a cell constituent derived from or located in mammals including humans. The biological material includes a collection of cells obtained from, derived from or in a tissue of the subject such as, for example, epithelium, connective tissue, blood vessels, muscle, nerve tissue, bone from any time in development of the subject. In various embodiments, the biological material includes healthy, diseased, or malignant tissue (e.g., cancerous or tumour tissue) comprising the target fluorophore (e.g., porphyrin). An example of the biological material further includes bacteria, including bacteria present in the subject (human, animal). Examples of the biological material which is a fluid include urine, serum, blood plasma, or blood. In various embodiments, the biological material may be a tissue section used in histochemistry, immunohistochemistry, cytochemistry, immunofluorescence, immunoblotting or other fluorescence-related imaging applications.

In various embodiments, the target fluorophore in the biological material is a fluorophore which when excited by a particular wavelength of light emits a light at a different, typically longer, wavelength. The target fluorophore includes a fluorophore which is of analytical, prognostic, diagnostic, physiological, pathological interest or a combination thereof. In various embodiments, the target fluorophore may be naturally occurring in the biological material (i.e., an endogenous fluorophore), externally administered into the biological material (i.e., an exogenous fluorophore) in a precursor or final form, or a combination thereof. Examples of naturally occurring or endogenous fluorophores include porphyrins, nicotinamide adenine dinucleotide (NAD), elastin, collagen, flavins, and amino acids. In embodiments where a porphyrin is the target fluorophore, the porphyrin includes a class of organic compounds that are in relevant biological systems and are formed as precursor intermediates in the biosynthesis of heme. For example, in humans and other mammals, porphyrins with 8-, 7-, 6-, 5- and 4-carboxyl groups are commonly formed in excess for heme synthesis, and thus are excreted in urine. In various embodiments, the term "porphyrin" includes, for example, porphyrin derivatives, coproporphyrin, uroporphyrin, protoporphyrin, porphyrin conjugates, liposomes, and nanovesicles.

Examples of exogenous fluorophores include various fluorescent probes or fluorescence inducing agents which may be used to augment (e.g., enhance) or provide fluorescent properties to a component of the biological material. For example, a fluorescent probe may associate with or attach to the component of the biological material to, for example, enhance fluorescence of an endogenous fluorophore in the component. Examples of exogenous fluorescent probes include fluorescein isothiocyanate (FITC), fluorescein, a fluorescent dye, 4',6-diaminidino-2-phenylindole (DAPI), and eosin. An example of a fluorescence inducing agent includes a gene which may be inserted into a cell chromosome to induce the production of fluorescent proteins (e.g., green fluorescent protein). The fluorescence inducing agent may be an adjuvant that can augment the fluorescence response of the target fluorophore. For example, in embodiments where the target fluorophore is porphyrin, the adjuvant may be a selected food source (e.g., porphyrinogenic foods or chemicals), aminolevulinic acid or inhibitors of certain enzymes in the HEME pathway (e.g., ferrochelatease inhibitors) which when consumed or administered to the subject, increase the fluorescence response of porphyrin.

The biological material naturally fluoresces or "autofluoresces" in the absence of exogenously administered fluorophores due to the presence of various endogenous fluorophores in the biological material. Autofluorescence originates from various fluorophores in the biological material, including for example nicotinamide adenine dinucleotide (NAD), elastin, collagen, flavins, amino acids, lipofuscins, advanced glycation end-products, and porphyrins. The biological material includes a material that has been processed or otherwise treated prior to being used in the various embodiments of the method and system of the invention. For example, in certain embodiments, pre-treatment may involve photobleaching of the biological material to reduce the autofluorescence of the biological material presumably by inactivating some of the autofluorescent endogenous fluorophores, and thus facilitating clearer subsequent resolution of the target fluorophore in cases where the target fluorophore is comparatively less susceptible to photobleaching or photobleaches at a slower rate than autofluorescent fluorophores in the biological material.

In accordance with the various embodiments, the method comprises illuminating the biological material with a first excitation light to induce a first fluorescence emission arising from both autofluorescence of the biological material and fluorescence of the target fluorophore, and with a second excitation light to induce a second fluorescence emission arising from the autofluorescence of the biological material. In various embodiments, the wavelength of the first excitation light is selected such that when the first excitation light illuminates the biological material, the fluorophores in the biological material which give rise to autofluorescence and the target fluorophore are both excited and emit a first fluorescence emission. In various embodiments, the wavelength of the second excitation light is selected such that only the fluorophores in the biological material giving rise to autofluorescence are excited and emit a second fluorescence emission. In various embodiments, for example, the first excitation light may have a wavelength ranging from about 350 nm to about 450 nm and the second excitation light may have a wavelength ranging from about 450 nm to about 700 nm. Illumination of the biological material with the first excitation light and the second excitation light includes intermittent illumination, continuous illumination or a combination thereof.

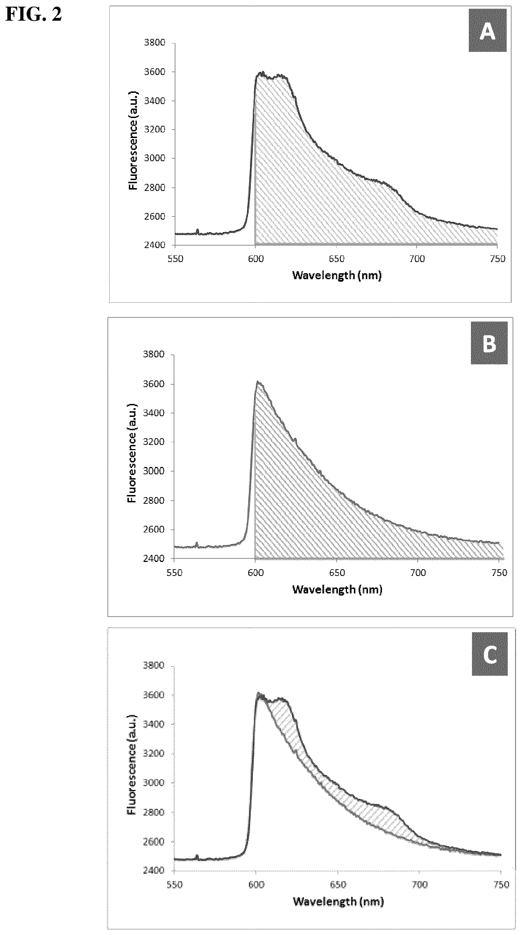

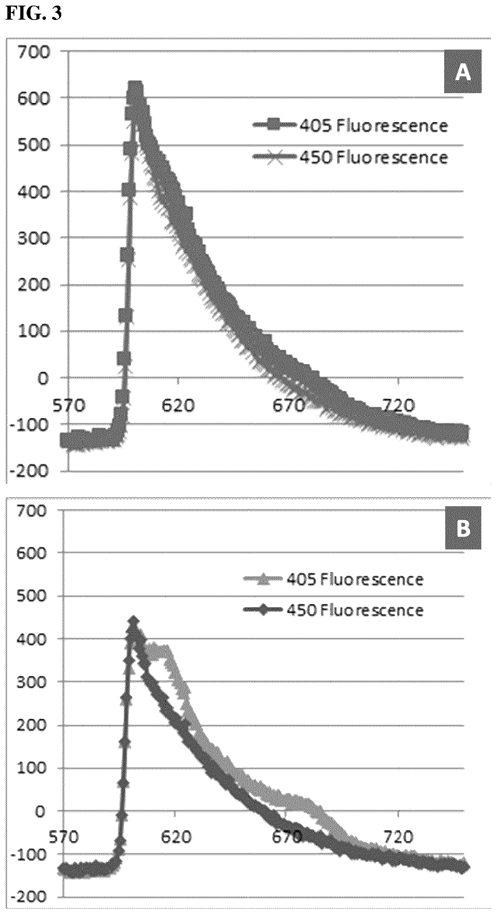

In the embodiment where the target fluorophore is porphyrin, the first excitation light has a wavelength of about 405 nm, and the second excitation light has a wavelength of about 450 nm. Figure **2** (shaded areas) illustrates data obtained from porphyrin in urine. In particular, Figure **2A** is a first fluorescence emission spectrum arising from both autofluorescence and porphyrin in urine, and Figure **2B** is a second fluorescence emission spectrum arising from autofluorescence only. Figure **2C** is the differential spectrum corresponding to porphyrin only. In the examples illustrated in Figure **2**, the urine was pretreated by photobleaching to facilitate a better discrimination of porphyrin from autofluorescence. In particular, photobleaching pre-treatment was conducted by illuminating the urine with the second excitation light of a wavelength of about 450 nm for about 3 minutes, which resulted in improved discrimination of the phorphyrin from autofluorescence of urine as compared with untreated urine (Figure **3**). Figure **3** shows fluorescence spectra from freshly obtained urine (Figure **3A**) and spectra obtained following an approximately 3-minute photobleaching exposure of the urine to light at about 450 nm (Figure **3B**).

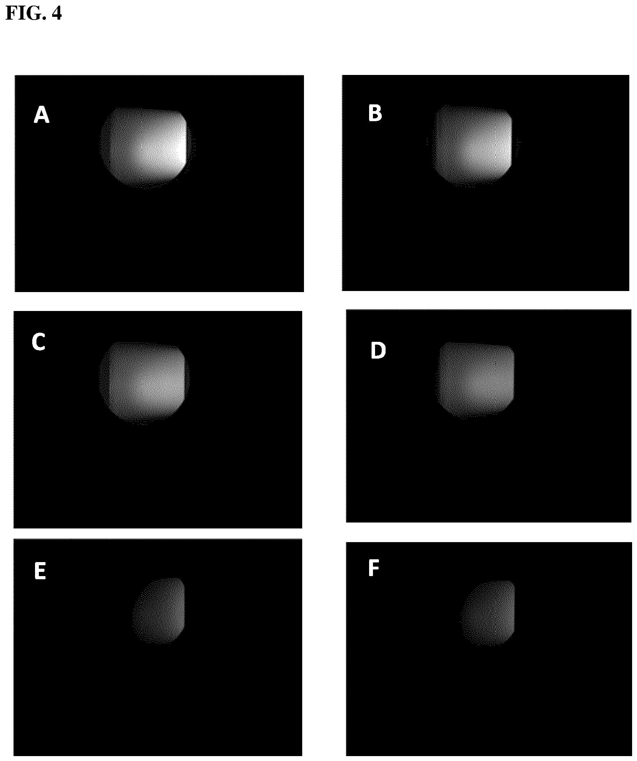

The method comprises acquiring a first fluorescence image from the first fluorescence emission and a second fluorescence image from the second fluorescence emission, and processing the first and second fluorescence images to extract a third fluorescence image representing the target fluorophore wherein the relative intensities of the first and second excitation light are modulated prior to acquiring the first and second fluorescence images. Figures **4A** to **4D** are images corresponding to the spectra in Figure **3** acquired when the urine is freshly obtained and when the urine has been exposed to light at about 450 nm for about 3 minutes. Figures **4E** and **4F** are the differential images corresponding to porphyrin only resulting from the processing as described in connection with the various embodiments.

Modulation of the relative intensities prior to image acquisition comprises identifying a wavelength region in the first and second fluorescence emissions, wherein the wavelength region is a region where emission arising from the fluorophore is present in the first fluorescence emission and absent in the second fluorescence emission, selecting a waveband outside the wavelength region, calculating at the selected waveband a ratio of relative intensities of the first and second fluorescence emissions, and adjusting the relative intensities of the first and second excitation lights to adjust the corresponding first fluorescence emission, second fluorescence emission or both until a suitable calculated ratio is achieved. According to various embodiments a waveband includes a wavelength. For example, in the embodiments where the target fluorophore is porphyrin, as is shown in Figure **2C** or Figure **3B**, the wavelength region where the emission arising from porphyrin is present in the first fluorescence emission and absent in the second fluorescence emission ranges, for example, from about 615 nm to about 625 nm and from about 660 nm to about 700 nm. Therefore, 600 nm was selected as the waveband outside this wavelength region and used as the waveband at which the ratio of the relative intensities at 405 nm and 450 nm was calculated for determining whether adjustment of the relative intensities is needed. In this example, the relative intensities were adjusted until the calculated ratio of about 1 was achieved within +/- 2%. In this example, the ratio was calculated at the 600 nm waveband by dividing an area-under-the curve value corresponding to the first fluorescence emission (i.e., the emission arising from excitation at about 405 nm) by an area-under-the curve value corresponding to the second fluorescence emission (i.e., the emission arising from excitation at about 450 nm). In various embodiments, the ratio may be calculated by dividing the intensity at the selected waveband (e.g., a selected wavelength) of the first fluorescence emission by the intensity at the selected waveband (e.g., a selected wavelength) of the second fluorescence emission. In various embodiments, other methods may be used for calculation of the ratio. For example, one or more intensity points in the spectra arising from the respective emissions at 405 nm and 450 nm at the selected waveband (e.g., 600 nm) rather than areas may be used for such a calculation.

Processing comprises subtracting the second fluorescence image from the first fluorescence image to produce an autofluorescence-free image of the target fluorophore (e.g., Figures **4E** and **4F**).

The methods and systems according to the various embodiments may be used for detecting *in situ* fluorescence. Experimental data in Figures **5** and **6** illustrate example results where porphyrin was applied topically on the skin of a subject. In this example, a porphyrin solution was prepared by dissolving about 0.1 mg of coproporphyrin ester (Sigma-Aldrich) in about 10 mL of dimethyl sulfoxide (DMSO, Sigma Aldrich). The porphyrin solution was applied onto a small area of the subject's forearm using a Q-tip. The fluorescence imaging system used to acquire the data featured a dual-excitation capability at the porphyrin absorption maxima of about 405 nm and about 450 nm. The latter was chosen as the shortest wavelength outside the main porphyrin absorption band, and due to its property to induce high levels of tissue autofluorescence. To ensure that the reflected excitation light does not interfere with the fluorescence images, a 600 nm band pass filter (600 nm ± 5 nm) was placed in front of the detector in the imaging system, and the excitation intensities at 405 nm and 450 nm were modulated until the ratio of autofluorescence at 450 nm to autofluorescence at 405 nm reached about 1. FIG. **5A** is an *in vivo* fluorescence image of the subject's forearm when excited with 405 nm light displaying autofluorescence from the forearm and porphyrin fluorescence. FIG. **5B** is a fluorescence image of the same region of the forearm as in FIG. **5A** upon excitation with 450 nm light. Since the autofluorescence induced by 450 nm excitation is greater than the autofluorescence induced by 405 nm excitation, the excitation light at 450 nm was modulated to produce autofluorescence at a level similar to the autofluorescence level in FIG **5A**. FIG. **5C** illustrates a fluorescence image of porphyrin with the autofluorescence removed in accordance with an embodiment.

Figure **5A** illustrates that the use of single excitation at 405 nm produces a well-localized fluorescence region arising from porphyrin fluorescence. High levels of background in surrounding areas arise from the presence of several endogenous fluorophores in skin (e.g., flavin adenine dinucleotide (FAD), nicotinamide adenine dinucleotide (NAD) and lipids). A similar autofluorescence pattern can also be observed when the same region was excited with different excitation wavelength (450 nm) away from the absorption spectra of porphyrins (Figure **5B**). Figure **5C** shows the resultant image after the processing according to the methods of the present invention where the autofluorescence was successfully attenuated using the method.

FIG. **6A** illustrates background intensity values at 405 nm and 450 nm after the excitation intensities were adjusted at 600 nm, and a lower than about 2% difference between background values was observed between excitations. FIG. **6B** illustrates a comparison of the signal-to-noise ratio (S/N Ratio) from fluorescence images excited at 405 nm and after background was removed in accordance with an embodiment. An increase in the S/N Ratio can be achieved using this approach as compared to using single excitation at 405 nm (see Figure **6B** where S/N Ratios of about 28.5 and about 1.9, respectively, are shown).

Various conventional approaches involve simultaneous acquisition of the fluorescence images where all fluorescence species are illuminated, and their fluorescence emissions are collected at the same time. The acquired images are then processed using one or more autofluorescence removal models involving spectral unmixing or background subtraction. Although various conventional approaches have been proposed for removing autofluorescence such, as for example, spectral unmixing (linear decomposition) and digital background subtraction to reveal the differential, such approaches rely on manipulating the images post-acquisition and pre-characterized spectra for autofluorescence, require calibration, and are susceptible to changes in sensitivity based on the concentration of the target fluorophore. While these methods may be cost effective and applicable to both *in vitro* and *in vivo* imaging, they are not able to completely remove the autofluorescence component from fluorescence images, and further to account for an instrumental background signal. The modulation of relative intensities of the first and second excitation lights prior to image acquisition, as described in connection with the various embodiments, compensates for relative changes in emission with time due to, for example, different rates of photobleaching between the target fluorophore (e.g., porphyrin) and the fluorophores in the biological material giving rise to autofluorescence. If the modulation of intensities is performed digitally post-image acquisition, as is described in the prior art, the accuracy of the processing of the two images to derive the image of the target fluorophore (e.g., subtraction) is decreased, especially if the magnitude of the first and second fluorescence signals is significantly different. Prior art spectral unmixing methods often require prior knowledge of the amount of autofluorescence in the sample, which may not be constant. In addition, images of the biological material may also include a certain amount of noise or background contributed by the acquisition system itself. Therefore, in contrast to the present invention, normalization of intensities post-image acquisition, as taught in the prior art, is noisier and limited in signal quality especially when the target fluorophore has a low level signal as compared to the autofluorescence signal (e.g., endogenous fluorophores or fluorophores in low-concentration components of the biological material). Furthermore, post-image acquisition amplification of the low level signal of the target fluorophore, as taught in the prior art, also amplifies the instrumental background signal, which further negatively impacts the signal quality. Unlike the prior art approaches, the present invention facilitates dynamic real-time correction for changes in fluorescence in the biological material, and therefore enables a real time representation of the nature of the biological material.

The data generated according to the various embodiments demonstrates that the dual-excitation method of the present invention, as described in connection with the various embodiments, facilitates a reduction in or mitigates the fluorescence background signal during fluorescence imaging of biological tissue by modulating the autofluorescence intensities at a selected wavelength prior to acquisition of fluorescence images. According to the various embodiments, acquisition of spectral images is carried out by timed excitation and light collection from only a target fluorophore of interest or background at a time. This temporal separation of excitation and fluorescence collection minimizes cross-talk. Instead of collecting the emission signal under the same excitation source, the present method according to the various embodiments, induces equivalent background levels by means of a second excitation wavelength (which does not induce fluorescence from the target fluorophore of interest), and then can be subsequently subtracted without decreasing the fluorescence signal from the target fluorophore of interest.

The present method can be beneficial for fluorescence imaging applications where tissue autofluorescence affects fluorescence imaging. The detection of equivalent autofluorescence signatures from different excitation sources facilitates a more accurate molecular diagnosis than a single fluorescence excitation. Moreover, the dual fluorescence imaging approach in accordance with the various embodiments is more robust and accurate than other post-processing analysis techniques since the fluorescence intensity of the fluorophore of interest is not affected by digitally removing the background or modulating the background levels. As is illustrated by the experimental data collected according to an embodiment, this method may be used for the identification of malignant tissues *in vivo* by exploiting the preferential accumulation of fluorophores such as porphyrins.

In accordance with an aspect of the invention, there is provided a system for extracting an image of a target fluorophore in a biological material. The system comprises means for illuminating the biological material with a first excitation light to induce a first fluorescence emission arising from both autofluorescence of the biological material and fluorescence of the target fluorophore and with a second excitation light to induce a second fluorescence emission arising from the autofluorescence of the biological material, means for acquiring a first fluorescence image from the first fluorescence emission and a second fluorescence image from the second fluorescence emission, means for modulating relative intensities of the first and second excitation lights prior to acquisition of the first and second fluorescence images, and means for processing the first and second fluorescence images to extract a third fluorescence image representing the target fluorophore.

Selected aspects relating to the system have been described above in connection with the various embodiments of the method of the present invention. Referring to Figure **7**, there is shown a system **10** for extracting the image of the target fluorophore **15** in the biological material **14.** The system **10** comprises the means for illuminating **12** for illumination (e.g., a light source configured to illuminate the biological material) of the biological material **14** with dual fluorescence excitation light, means for acquiring **16** fluorescence images (e.g., an image acquisition assembly configured to acquire fluorescence images) arising from both the autofluorescence and the target fluorophore and from the autofluorescence alone, and means for processing **18** the acquired fluorescence images (e.g., a processor assembly configured to process the acquired images) to extract an image representing only the target fluorophore. In various embodiments, the means for illuminating **12** comprises, for example, an illumination module **20** shown in Figure **8****.** The illumination module **20** comprises a fluorescence excitation source **22** operatively configured for providing fluorescence excitation having suitable intensities and suitable wavelengths for exciting the target fluorophore and the fluorophores giving rise to autofluorescence. In one embodiment, the fluorescence excitation source **22** may be a single excitation source having dual excitation capabilities for providing a first excitation light for inducing emission arising from both autofluorescence and fluorescence of the target fluorophore, and the second excitation light for inducing emission arising from the autofluoresence only. In another embodiment, the fluorescence excitation source **22** may comprise two excitation sources (not shown), one for providing the first excitation light and the other for providing the second excitation light. In various embodiments, the fluorescence excitation source **22** includes, for example, a laser diode (which may comprise, for example, one or more fiber-coupled diode lasers), one or more LEDs, arc lamps, or other illuminant technologies of sufficient intensity and appropriate wavelength for providing the first and second excitation lights. In various embodiments, the first and second excitation light from the fluorescence excitation source **22** may be projected through an optical element (i.e., one or more optical elements) to shape and guide the output being used to illuminate the biological sample. The shaping optical element may consist, for example, of one or more lenses, light guides and diffusers. As is illustrated in Figure **8**, the output **24** from the fluorescence excitation source **22** is passed through one or more focusing lenses **26**, and then through a homogenizing light pipe **28** such as, for example, light pipes commonly available from Newport Corporation, USA. Finally, the light is passed through an optical diffuser **30** (i.e., one or more optical diffusers or diffractive elements) such as, for example, ground glass diffusers also available from Newport Corporation, USA. Power to the fluorescence excitation source **22** itself is provided by, for example, a high-current laser driver such as those available from Lumina Power Inc., USA. In the embodiment where the fluorescence excitation source **22** is a laser, the laser may be operated in a pulsed mode during the image acquisition process. In this embodiment, an optical sensor such as a solid state photodiode **32** is incorporated into the illumination module **20** and samples the illumination intensity produced by the illumination module **20** *via* scattered or defuse reflections from the various optical elements.

In an alternative embodiment, the means for illuminating **12** may also be configured to provide an additional functionality such as white light illumination. In another embodiment, the method and system of the present invention may further comprise acquiring and combining the third fluorescence image representing the target fluorophore with a white light image of the biological material. In this manner, the location of the targeted fluorophore can be visualized within the context of the biological material. This is useful in instances in which the biological material cannot be viewed directly with the human eye.

In various embodiments, the illumination module **20** in Figure **8** comprises means for modulating (not shown) the relative intensities of the first and second excitation lights from the fluorescence excitation source **22**, so as to allow intensity adjustment. Such means for modulation may include modulation of the power to the light source, mechanical interruption of the light beam by shutters, apertures or choppers, optical, opto-mechanical or electro-optical diversion, filtering or blocking of the light beam or similar means.

Referring back to Figure **7**, the means for acquiring **16** comprises, for example, a fluorescence emission acquisition module **30** (e.g., a camera module) shown in Figure **9** for acquiring the first and second fluorescence images. As is shown in Figure **9**, the fluorescence emission **42** from the target fluorophore in the biological material and the fluorescence emission from other fluorophores giving rise to autofluorescence or both is collected and focused onto an image sensor **44** using an arrangement of various optical elements, e.g., **46a**, **46b**, **48** and **50.** The charge that results from the optical signal transduced by the image sensor **44** is converted to a video signal by the appropriate read-out and amplification electronics in the fluorescence emission acquisition module **30.**

Referring back to Figure **7**, in various embodiments, the means for processing **18** (e.g., a processor assembly) comprises, for example, a processor module (not shown) for analyzing the emission signals, performing calculations for subtracting the second fluorescence image from the first fluorescence image to output the calculated information to an appropriate display and/or recording device. In various embodiments, the processor module comprises any computer or computing means such as, for example, a tablet, laptop, desktop or networked computer. In various embodiments, the processor module may have a data storage module with the capability to save data (e.g., image sequences) to a tangible non-transitory computer readable medium such as, for example, internal memory, a hard disk, or flash memory, so as to enable recording and/or post-processing of acquired data. In various embodiments, the processor module may have an internal clock to enable control of the various elements and ensure correct timing of illumination and sensor shutters. In various other embodiments, the processor module may also provide user input and graphical display of outputs. The imaging system may optionally be configured with a video display (not shown) to display the images as they are being acquired or played back after recording, or further to visualize the data generated at various stages of the method. In various embodiments, the means for processing is in communication with an imaging system or is a component of the imaging system. An example of the imaging system in accordance with an embodiment is an endoscope.

In operation, and with continuing reference to the embodiments in Figures **7** to **9**, the biological material is positioned in the illumination path of the means for illuminating **12** of the system **10** comprising the illumination module **20**, and such that, for example, the illumination module **20** produces a substantially uniform field of illumination across substantially the entire area of the biological material. The fluorescence excitation source **22** (e.g., the laser diode) is turned on and begins the shutter sequence for the image sensor (e.g., image sensor **44** of the fluorescence emission acquisition module **30**). The fluorescence emission from the biological material is collected by the front imaging optics of the fluorescence emission acquisition module **30** such as optics **46a** for example in Figure **9** at the selected waveband (e.g., for porphyrin the selected wavelength is about 600 nm), and a ratio of the relative intensities is calculated. If the calculated ratio is suitable (e.g., for porphyrin, a suitable calculated ratio is in the range of about 0.98 to 1.02), the first and second fluorescence images are acquired. If the ratio is not suitable, the relative intensities of the first and second excitation lights are modulated and the ratio is re-calculated until a suitable ratio is achieved. The obtained first and second fluorescence images are then subtracted to extract a third fluorescence image representing only the target fluorophore.

According to another aspect of the disclosure there is provided a tangible non-transitory computer readable medium having computer-executable (readable) program code means embedded thereon comprising a method for extracting an image of a target fluorophore in a biological material wherein a waveband for the target fluorophore emission overlaps a waveband for autofluorescence emission in the biological material, the method comprising:
illuminating the biological material with a first excitation light to induce a first fluorescence emission arising from both autofluorescence of the biological material and fluorescence of the target fluorophore and with a second excitation light to induce a second fluorescence emission arising from the autofluorescence of the biological material;
acquiring a first fluorescence image from the first fluorescence emission and a second fluorescence image from the second fluorescence emission; and
processing the first and second fluorescence images to extract a third fluorescence image representing the target fluorophore, wherein relative intensities of the first and second excitation lights are modulated prior to acquiring the first and second fluorescence images.

One skilled in the art will appreciate that program code means can be written in any appropriate programming language and delivered to the processor in many forms, including, for example, but not limited to information permanently stored on non-writeable storage media (e.g., read-only memory devices such as ROMs or CD-ROM disks), information alterably stored on writeable storage media (e.g., hard drives), information conveyed to the processor through communication media, such as a local area network, a public network such as the Internet, or any type of media suitable for storing electronic instruction. When carrying computer readable instructions such computer readable media represent examples of various implementations of the present disclosure. In various examples, the tangible non-transitory computer readable medium comprises all computer-readable media, and the present disclosure is limited to computer readable media wherein the media is both tangible and non-transitory.

In yet further aspects, there is provided a kit including the system and the exogenous fluorophore as described in connection with the various embodiments.

Therefore, the various embodiments of the invention facilitate discrimination of the fluorescence of interest from an unknown combination of autofluorescence and fluorescence of interest. The present invention facilitates improvements in image quality for target fluorophores, preserves signal fluorescence while eliminating autofluorescence as well as background, and increases the resulting signal to autofluorescence ratio and the overall sensitivity of detection. The present invention is adaptable to a wide array of biological materials, and may be applied to any fluorescence imaging application. The present invention may be used to image and analyze a biological sample to discern the presence, absence, concentration, and/or spatial distribution of one of more fluorophore targets in the biological material. The present invention may be further used as a complementary tool for medical assessment or biological assessment (e.g., assessment of a biological phenomenon), diagnostic assessment, therapeutic assessment, physiological assessment, or a combination thereof.

While the present invention has been illustrated and described in connection with various embodiments shown and described in detail, it is not intended to be limited to the details shown, since various modifications and structural changes may be made without departing in any way from the scope of the present invention. Various modifications of form, arrangement of components, steps, details and order of operations of the embodiments illustrated, as well as other embodiments of the invention may be made without departing in any way from the scope of the present invention, and will be apparent to a person of skill in the art upon reference to this description. It is therefore contemplated that the appended claims will cover such modifications and embodiments as they fall within the true scope of the invention. For the terms "for example" and "such as," and grammatical equivalences thereof, the phrase "and without limitation" is understood to follow unless explicitly stated otherwise. As used herein, the singular forms "a," "an," and "the" include plural referents unless the context clearly dictates otherwise.

## Claims

1. A method for extracting an image of a target fluorophore in a biological material wherein a waveband for the target fluorophore emission overlaps a waveband for autofluorescence emission in the biological material, the method comprising:
illuminating the biological material with a first excitation light to induce a first fluorescence emission arising from both autofluorescence of the biological material and fluorescence of the target fluorophore and with a second excitation light to induce a second fluorescence emission arising only from the autofluorescence of the biological material;
acquiring a first fluorescence image from the first fluorescence emission and a second fluorescence image from the second fluorescence emission; and
processing the first and second fluorescence images to extract a third fluorescence image representing the target fluorophore,
wherein relative intensities of the first and second excitation lights with respect to each other are
modulated prior to acquiring the first and second fluorescence images, wherein the modulation of the relative intensities comprises:
identifying a wavelength region in the first and second fluorescence emissions, wherein the wavelength region is a region where emission arising from the fluorophore is present in the first fluorescence emission and absent in the second fluorescence emission;
selecting a waveband outside the wavelength region;
calculating at the selected waveband a ratio of relative intensities of the first and second fluorescence emissions with respect to each other; and
adjusting the relative intensities of the first and second excitation lights with respect to each other to adjust the corresponding first fluorescence emission, second fluorescence emission or both until a suitable calculated ratio is achieved.

2. The method of claim 1 wherein the waveband outside the wavelength region comprises one or more wavelengths in fluorescence spectra arising from the first and second fluorescence emissions.

3. The method of claim 1 or 2 wherein calculating the ratio of relative intensities of the first and second fluorescence emissions comprises dividing an area-under-the curve value corresponding to the first fluorescence emission by an area-under-the curve value corresponding to the second fluorescence emission.

4. The method of any one of claims 1 to 3 wherein the first excitation light has a wavelength of about 405 nm and the second excitation light has a wavelength of about 450 nm when the target fluorophore is porphyrin.

5. The method of any one of claims 1 to 4 wherein the selected waveband is about 600 nm and wherein the calculated ratio is about 1.

6. The method of any one of claims 1 to 5 wherein processing the first and second fluorescence images to extract the third fluorescence image representing the target fluorophore comprises subtracting the second fluorescence image from the first fluorescence image.

7. The method of any one of claims 1 to 6 wherein the biological material is pretreated by photobleaching.

8. The method of any one of claims 1 to 7 wherein the target fluorophore is endogenous, exogenous, or a combination thereof.

9. The method of claim 8 wherein the endogenous fluorophore is porphyrin, a porphyrin precursor, a porphyrin analog, a porphyrin derivative, a porphyrin conjugate, a porphyrin liposome, a porphyrin nanovesicle, or a combination thereof.

10. The method of claim 9 wherein the porphyrin comprises a coproporphyrin, a uroporphyrin, a protoporphyrin, or a combination thereof.

11. The method of claim 8 wherein the exogenous fluorophore is a fluorescent dye, a fluorescence inducing agent, or a combination thereof.

12. Use of the method of any one of claims 1 to 11 in hystochemistry, cytochemistry, or a combination thereof.

13. A system (10) for extracting an image of a target fluorophore (15) in a biological material (14) wherein a waveband for the target fluorophore emission overlaps a waveband for autofluorescence emission in the biological material, the system comprising:
means for illuminating (12) the biological material with a first excitation light to induce a first fluorescence emission arising from both autofluorescence of the biological material and fluorescence of the target fluorophore and with a second excitation light to induce a second fluorescence emission arising only from the autofluorescence of the biological material;
means for acquiring (16)
a first fluorescence image from the first fluorescence emission and a second fluorescence image from the second fluorescence emission;
means for modulating relative intensities of the first and second excitation lights with respect to each other prior to acquisition of the first and second fluorescence images; and
means for processing (18) the first and second fluorescence images to extract a third fluorescence image representing the target fluorophore, wherein the means for modulating the relative intensities comprises:
means for identifying a wavelength region in the first and second fluorescence emissions, wherein the wavelength region is a region where emission arising from the fluorophore is present in the first fluorescence emission and absent in the second fluorescence emission;
means for selecting a waveband outside the wavelength region;
means for calculating at the selected waveband a ratio of the relative intensities of the first and second fluorescence emissions with respect to each other; and
means for adjusting the relative intensities of the first and second excitation with respect to each other to adjust the corresponding first fluorescence emission,
second fluorescence emission or both until a suitable calculated ratio is achieved.

14. The system of claim 13 wherein the waveband outside the wavelength region comprises one or more wavelengths in fluorescence spectra arising from the first and second fluorescence emissions.

15. The system of claim 13 or 14 wherein means for calculating the ratio of relative intensities of the first and second fluorescence emissions comprises means for dividing an area-under-the curve value corresponding to the first fluorescence emission by an area-under-the curve value corresponding to the second fluorescence emission.

16. The system of any one of claims 13 to 15 wherein the first excitation light has a wavelength of about 405 nm and the second excitation light has a wavelength of about 450 nm when the target fluorophore is porphyrin.

17. The system of any one of claims 13 to 16 wherein the selected waveband is about 600 nm and wherein the calculated ratio is about 1.

18. The system of any one of claims 13 to 17 wherein the means for processing the first and second fluorescence images to extract the third fluorescence image representing the target fluorophore comprises means for subtracting the second fluorescence image from the first fluorescence image.

19. The system of any one of claims 13 to 18 wherein the means for illuminating comprises an illumination module comprising a fluorescence excitation source, the fluorescence excitation source operatively configured to generate the first and second excitation lights.

20. The system of claim 19 wherein the illumination module further comprises an optical element operatively configured to shape and guide the first and second excitation lights exiting the illumination module.

21. The system of claim 20 wherein the optical element comprises a lens, a light guide, a diffuser, or a combination thereof.

22. The system of any one of claims 13 to 21 wherein the means for acquiring comprises a fluorescence emission acquisition module, the fluorescence emission acquisition module comprising an image sensor.

23. The system of claim 22 wherein the fluorescence emission acquisition module further comprises an optical element disposed in front of the image sensor operatively configured to capture, filter, and direct the first and second fluorescence emissions.

24. The system of any one of claims 13 to 23 wherein the means for processing comprises a processor module.

25. The system of claim 24 wherein the processor module is operatively configured to control an operation of the means for illuminating, to control an operation of the means for acquiring, or a combination thereof.

26. The system of any one of claims 13 to 25 wherein the biological material is pretreated by photobleaching.

27. The system of any one of claims 13 to 26 wherein the target fluorophore is endogenous, exogenous, or a combination thereof.

28. The system of claim 27 wherein the endogenous fluorophore is porphyrin, a porphyrin precursor, a porphyrin analog, a porphyrin derivative, a porphyrin conjugate, a porphyrin liposome, a porphyrin nanovesicle, or a combination thereof.

29. The system of claim 28 wherein the porphyrin comprises a coproporphyrin, a uroporphyrin, a protoporphyrin, or a combination thereof.

30. The system of claim 27 wherein the exogenous fluorophore is a fluorescent dye, a fluorescence inducing agent, or a combination thereof.

31. The method of any one of claims 1 to 11, the use of claim 12, or the system of any one of claims 13 to 30 wherein the biological material comprises a biological tissue, a biological fluid, or a fraction thereof.

32. The method of any one of claims 1 to 11, the use of claim 12, or the system of any one of claims 13 to 30 wherein the biological material comprises an organ, a cell, a cell line, a cell constituent derived from or located in a mammal.

33. The method of any one of claims 1 to 11, the use of claim 12, or the system of any one of claims 13 to 30 wherein the biological material comprises healthy, diseased or malignant tissue.

34. The method of any one of claims 1 to 11, the use of claim 12, or the system of any one of claims 13 to 30 wherein the biological material comprises a tissue section for use in histochemistry, immunohistochemistry, cytochemistry, immunofluorescence, immunoblotting, or a fluorescence-related imaging application.

## Patentansprüche

1. Verfahren zum Extrahieren eines Bildes eines Zielfluorophors in einem biologischen Material, wobei ein Wellenband für die Zielfluorophoremission ein Wellenband für die Autofluoreszenzemission in dem biologischen Material überlappt, wobei das Verfahren umfasst:
Beleuchten des biologischen Materials mit einem ersten Anregungslicht, um eine erste Fluoreszenzemission zu induzieren, die sowohl aus der Autofluoreszenz des biologischen Materials als auch aus der Fluoreszenz des Zielfluorophors entsteht, und mit einem zweiten Anregungslicht, um eine zweite Fluoreszenzemission zu induzieren, die nur aus der Autofluoreszenz des biologischen Materials entsteht;
Erfassen eines ersten Fluoreszenzbildes von der ersten Fluoreszenzemission und eines zweiten Fluoreszenzbildes von der zweiten Fluoreszenzemission; und
Verarbeiten des ersten und zweiten Fluoreszenzbildes, um ein drittes Fluoreszenzbild zu extrahieren, das das Zielfluorophor darstellt;
wobei die relativen Intensitäten des ersten und des zweiten Anregungslichts in Bezug aufeinander vor dem Erfassen des ersten und des zweiten Fluoreszenzbildes moduliert werden, wobei
die Modulation der relativen Intensitäten umfasst:
Identifizieren eines Wellenlängenbereichs in der ersten und zweiten Fluoreszenzemission, wobei der Wellenlängenbereich ein Bereich ist, in dem die vom Fluorophor entstehende Emission in der ersten Fluoreszenzemission vorhanden ist und in der zweiten Fluoreszenzemission fehlt;
Auswählen eines Wellenbandes außerhalb des Wellenlängenbereichs;
Berechnen eines Verhältnisses der relativen Intensitäten der ersten und zweiten Fluoreszenzemissionen in Bezug aufeinander im ausgewählten Wellenbereich; und
Anpassen der relativen Intensitäten des ersten und des zweiten Anregungslichts in Bezug aufeinander, um die entsprechende erste Fluoreszenzemission, zweite Fluoreszenzemission oder beide anzupassen, bis ein geeignetes berechnetes Verhältnis erreicht ist.

2. Verfahren nach Anspruch 1, wobei das Wellenband außerhalb des Wellenlängenbereichs eine oder mehrere Wellenlängen in Fluoreszenzspektren umfasst, die aus der ersten und zweiten Fluoreszenzemission entstehen.

3. Verfahren nach Anspruch 1 oder 2, wobei das Berechnen des Verhältnisses der relativen Intensitäten der ersten und zweiten Fluoreszenzemission das Teilen eines Bereichs unter dem Kurvenwert, der der ersten Fluoreszenzemission entspricht, durch einen Bereich unter dem Kurvenwert, der der zweiten Fluoreszenzemission entspricht, umfasst.

4. Verfahren nach einem der Ansprüche 1 bis 3, wobei das erste Anregungslicht eine Wellenlänge von etwa 405 nm und das zweite Anregungslicht eine Wellenlänge von etwa 450 nm hat, wenn das Zielfluorophor Porphyrin ist.

5. Verfahren nach einem der Ansprüche 1 bis 4, wobei das ausgewählte Wellenband etwa 600 nm beträgt, und wobei das berechnete Verhältnis etwa 1 beträgt.

6. Verfahren nach einem der Ansprüche 1 bis 5, wobei das Verarbeiten des ersten und des zweiten Fluoreszenzbildes zum Extrahieren des dritten Fluoreszenzbildes, das das Zielfluorophor darstellt, das Subtrahieren des zweiten Fluoreszenzbildes von dem ersten Fluoreszenzbild umfasst.

7. Verfahren nach einem der Ansprüche 1 bis 6, wobei das biologische Material durch Photobleichen vorbehandelt wird.

8. Verfahren nach einem der Ansprüche 1 bis 7, wobei das Zielfluorophor endogen, exogen oder eine Kombination davon ist.

9. Verfahren nach Anspruch 8, wobei das endogene Fluorophor Porphyrin, ein Porphyrin-Vorläufer, ein Porphyrin-Analogon, ein Porphyrin-Derivat, ein Porphyrin-Konjugat, ein Porphyrin-Liposom, ein Porphyrin-Nanovesikel oder eine Kombination davon ist.

10. Verfahren nach Anspruch 9, wobei das Porphyrin ein Coproporphyrin, ein Uroporphyrin, ein Protoporphyrin oder eine Kombination davon umfasst.

11. Verfahren nach Anspruch 8, wobei das exogene Fluorophor ein fluoreszierender Farbstoff, ein fluoreszenzinduzierendes Mittel oder eine Kombination davon ist.

12. Verwendung des Verfahrens nach einem der Ansprüche 1 bis 11 in der Hystochemie, Zytochemie oder einer Kombination davon.

13. System (10) zum Extrahieren eines Bildes eines Zielfluorophors (15) in einem biologischen Material (14), wobei ein Wellenband für die Zielfluorophoremission ein Wellenband für die Autofluoreszenzemission in dem biologischen Material überlappt, wobei das System umfasst:
Mittel zum Beleuchten (12) des biologischen Materials mit einem ersten Anregungslicht, um eine erste Fluoreszenzemission zu induzieren, die sowohl aus der Autofluoreszenz des biologischen Materials als auch aus der Fluoreszenz des Zielfluorophors entsteht, und mit einem zweiten Anregungslicht, um eine zweite Fluoreszenzemission zu induzieren, die nur aus dem Autofluoreszenz des biologischen Materials entsteht;
Mittel zum Erfassen (1) eines ersten Fluoreszenzbildes von der ersten Fluoreszenzemission und eines zweiten Fluoreszenzbildes von der zweiten Fluoreszenzemission;
Mittel zum Modulieren der relativen Intensitäten des ersten und des zweiten Anregungslichts in Bezug aufeinander vor der Aufnahme des ersten und des zweiten Fluoreszenzbildes; und
Mittel zum Verarbeiten (18) des ersten und zweiten Fluoreszenzbildes zum Extrahieren eines dritten Fluoreszenzbildes, das das Zielfluorophor darstellt, wobei das Mittel zum Modulieren der relativen Intensitäten umfasst:
Mittel zum Identifizieren eines Wellenlängenbereichs in der ersten und zweiten Fluoreszenzemission, wobei der Wellenlängenbereich ein Bereich ist, in dem die vom Fluorophor entstehende Emission in der ersten Fluoreszenzemission vorhanden ist und in der zweiten Fluoreszenzemission fehlt;
Mittel zum Auswählen eines Wellenbandes außerhalb des Wellenlängenbereichs;
Mittel zum Berechnen eines Verhältnisses der relativen Intensitäten der ersten und zweiten Fluoreszenzemission in Bezug aufeinander im ausgewählten Wellenbereich; und
Mittel zum Anpassen der relativen Intensitäten der ersten und zweiten Anregung in Bezug aufeinander, um die entsprechende erste Fluoreszenzemission, zweite Fluoreszenzemission oder beides anzupassen, bis ein geeignetes berechnetes Verhältnis erreicht ist.

14. System nach Anspruch 13, wobei das Wellenband außerhalb des Wellenlängenbereichs eine oder mehrere Wellenlängen in Fluoreszenzspektren umfasst, die aus der ersten und zweiten Fluoreszenzemission entstehen.

15. System nach Anspruch 13 oder 14, wobei Mittel zum Berechnen des Verhältnisses der relativen Intensitäten der ersten und zweiten Fluoreszenzemission Mittel zum Teilen eines Bereichs unter dem Kurvenwert, der der ersten Fluoreszenzemission entspricht, durch einen Bereich unter dem Kurvenwert, der der zweiten Fluoreszenzemission entspricht, umfasst.

16. System nach einem der Ansprüche 13 bis 15, wobei das erste Anregungslicht eine Wellenlänge von etwa 405 nm und das zweite Anregungslicht eine Wellenlänge von etwa 450 nm hat, wenn das Zielfluorophor Porphyrin ist.

17. System nach einem der Ansprüche 13 bis 16, wobei das ausgewählte Wellenband etwa 600 nm beträgt und wobei das berechnete Verhältnis ungefähr 1 beträgt.

18. System nach einem der Ansprüche 13 bis 17, wobei das Mittel zum Verarbeiten des ersten und des zweiten Fluoreszenzbildes zum Extrahieren des dritten Fluoreszenzbildes, das das Zielfluorophor darstellt, Mittel zum Subtrahieren des zweiten Fluoreszenzbildes vom ersten Fluoreszenzbild umfasst.

19. System nach einem der Ansprüche 13 bis 18, wobei das Mittel zum Beleuchten ein Beleuchtungsmodul umfasst, das eine Fluoreszenzanregungsquelle umfasst, wobei die Fluoreszenzanregungsquelle betriebsmäßig konfiguriert ist, um das erste und das zweite Anregungslicht zu erzeugen.

20. System nach Anspruch 19, wobei das Beleuchtungsmodul ferner ein optisches Element umfasst, das betriebsmäßig konfiguriert ist, um das erste und das zweite Anregungslicht, das aus dem Beleuchtungsmodul austritt, zu formen und zu führen.

21. System nach Anspruch 20, wobei das optische Element eine Linse, einen Lichtleiter, einen Diffusor oder eine Kombination davon umfasst.

22. System nach einem der Ansprüche 13 bis 21, wobei das Mittel zum Erfassen ein Fluoreszenzemissionserfassungsmodul umfasst, wobei das Fluoreszenzemissionserfassungsmodul einen Bildsensor umfasst.

23. System nach Anspruch 22, wobei das Fluoreszenzemissionserfassungsmodul ferner ein optisches Element umfasst, das vor dem Bildsensor angeordnet ist, der betriebsmäßig konfiguriert ist, um die erste und zweite Fluoreszenzemission zu erfassen, zu filtern und zu lenken.

24. System nach einem der Ansprüche 13 bis 23, wobei das Mittel zur Verarbeitung ein Prozessormodul umfasst.

25. System nach Anspruch 24, wobei das Prozessormodul betriebsmäßig konfiguriert ist, um einen Betrieb des Mittels zum Beleuchten, einen Betrieb des Mittels zum Erfassen oder eine Kombination davon zu steuern.

26. System nach einem der Ansprüche 13 bis 25, wobei das biologische Material durch Photobleichen vorbehandelt wird.

27. System nach einem der Ansprüche 13 bis 26, wobei das Zielfluorophor endogen, exogen oder eine Kombination davon ist.

28. System nach Anspruch 27, wobei das endogene Fluorophor Porphyrin, ein Porphyrin-Vorläufer, ein Porphyrin-Analogon, ein Porphyrin-Derivat, ein Porphyrin-Konjugat, ein Porphyrin-Liposom, ein Porphyrin-Nanovesikel oder eine Kombination davon ist.

29. System nach Anspruch 28, wobei das Porphyrin ein Coproporphyrin, ein Uroporphyrin, ein Protoporphyrin oder eine Kombination davon umfasst.

30. System nach Anspruch 27, wobei das exogene Fluorophor ein fluoreszierender Farbstoff, ein fluoreszenzinduzierendes Mittel oder eine Kombination davon ist.

31. Verfahren nach einem der Ansprüche 1 bis 11, Verwendung nach Anspruch 12 oder System nach einem der Ansprüche 13 bis 30, wobei das biologische Material ein biologisches Gewebe, eine biologische Flüssigkeit oder einen Bruchteil davon umfasst.

32. Verfahren nach einem der Ansprüche 1 bis 11, Verwendung nach Anspruch 12 oder System nach einem der Ansprüche 13 bis 30, wobei das biologische Material ein Organ, eine Zelle, eine Zelllinie, einen Zellbestandteil umfasst, der von einem Säugetier abgeleitet ist oder sich darin befindet.

33. Verfahren nach einem der Ansprüche 1 bis 11, Verwendung nach Anspruch 12 oder System nach einem der Ansprüche 13 bis 30, wobei das biologische Material gesundes, krankes oder bösartiges Gewebe umfasst.

34. Verfahren nach einem der Ansprüche 1 bis 11, Verwendung nach Anspruch 12 oder System nach einem der Ansprüche 13 bis 30, wobei das biologische Material einen Gewebeschnitt zur Verwendung in der Histochemie, Immunhistochemie, Zytochemie, Immunfluoreszenz, Immunblotting oder einer fluoreszenzbezogenen Bildgebungsanwendung umfasst.

## Revendications

1. Méthode d'extraction d'une image d'un fluorophore cible dans une matière biologique dans laquelle une bande d'ondes pour l'émission d'une fluorophore cible chevauche une bande d'ondes pour une émission d'autofluorescence dans la matière biologique, la méthode comprenant :
l'éclairage de la matière biologique avec une première lumière d'excitation pour induire une première émission de fluorescence résultant à la fois d'une autofluorescence de la matière biologique et d'une fluorescence du fluorophore cible et avec une seconde lumière d'excitation pour induire une seconde émission de fluorescence résultant uniquement de l'autofluorescence de la matière biologique ;
l'acquisition d'une première image de fluorescence à partir de la première émission de fluorescence et d'une deuxième image de fluorescence à partir de la seconde émission de fluorescence ; et
le traitement des première et deuxième images de fluorescence pour extraire une troisième image de fluorescence représentant le fluorophore cible,
dans laquelle les intensités relatives des première et seconde lumières d'excitation l'une par rapport à l'autre sont modulées avant l'acquisition des première et deuxième images de fluorescence,
dans laquelle la modulation des intensités relatives comprend :
l'identification d'une région de longueurs d'onde dans les première et seconde émissions de fluorescence, dans laquelle la région de longueurs d'onde est une région où une émission résultant du fluorophore est présente dans la première émission de fluorescence et absente dans la seconde émission de fluorescence ;
la sélection d'une bande d'ondes hors de la région de longueurs d'onde ;
le calcul dans la bande d'ondes sélectionnée d'un rapport des intensités relatives des première et seconde émissions de fluorescence l'une par rapport à l'autre ; et
l'ajustement des intensités relatives des première et seconde lumières d'excitation l'une par rapport à l'autre pour ajuster la première émission de fluorescence correspondante, la seconde émission de fluorescence ou les deux jusqu'à ce qu'un rapport calculé approprié soit obtenu.

2. Méthode selon la revendication 1, dans laquelle la bande d'ondes hors de la région de longueurs d'onde comprend une ou plusieurs longueurs d'onde dans des spectres de fluorescence résultant des première et seconde émissions de fluorescence.

3. Méthode selon la revendication 1 ou 2, dans laquelle le calcul du rapport des intensités relatives des première et seconde émissions de fluorescence comprend la division d'une valeur d'aire sous la courbe correspondant à la première émission de fluorescence par une valeur d'aire sous la courbe correspondant à la seconde émission de fluorescence.

4. Méthode selon l'une quelconque des revendications 1 à 3, dans laquelle la première lumière d'excitation présente une longueur d'onde d'environ 405 nm et la seconde lumière d'excitation présente une longueur d'onde d'environ 450 nm quand le fluorophore cible est la porphyrine.

5. Méthode selon l'une quelconque des revendications 1 à 4, dans laquelle la bande d'ondes sélectionnée est d'environ 600 nm et dans laquelle le rapport calculé est d'environ 1.

6. Méthode selon l'une quelconque des revendications 1 à 5, dans laquelle le traitement des première et deuxième images de fluorescence pour extraire la troisième image de fluorescence représentant le fluorophore cible comprend la soustraction de la deuxième image de fluorescence de la première image de fluorescence.

7. Méthode selon l'une quelconque des revendications 1 à 6, dans laquelle la matière biologique est prétraitée par photoblanchiment.

8. Méthode selon l'une quelconque des revendications 1 à 7, dans laquelle le fluorophore cible est endogène, exogène ou une combinaison de ceux-ci.

9. Méthode selon la revendication 8, dans laquelle le fluorophore endogène est la porphyrine, un précurseur de porphyrine, un analogue de porphyrine, un dérivé de porphyrine, un conjugué de porphyrine, un liposome de porphyrine, une nanovésicule de porphyrine, ou une combinaison de ceux-ci.

10. Méthode selon la revendication 9, dans laquelle la porphyrine comprend une coproporphyrine, une uroporphyrine, une protoporphyrine, ou une combinaison de celles-ci.

11. Méthode selon la revendication 8, dans laquelle le fluorophore exogène est un colorant fluorescent, un agent induisant une fluorescence, ou une combinaison de ceux-ci.

12. Utilisation de la méthode selon l'une quelconque des revendications 1 à 11 en histochimie, cytochimie, ou une combinaison de celles-ci.

13. Système (10) d'extraction d'une image d'un fluorophore cible (15) dans une matière biologique (14)
dans lequel une bande d'ondes pour l'émission d'un fluorophore cible chevauche une bande d'ondes pour une émission d'autofluorescence dans la matière biologique, le système comprenant :
un moyen pour éclairer (12) la matière biologique avec une première lumière d'excitation pour induire une première émission de fluorescence résultant à la fois d'une autofluorescence de la matière biologique et d'une fluorescence du fluorophore cible et avec une seconde lumière d'excitation pour induire une seconde émission de fluorescence résultant uniquement de l'autofluorescence de la matière biologique ;
un moyen pour acquérir (16) une première image de fluorescence à partir de la première émission de fluorescence et une deuxième image de fluorescence à partir de la seconde émission de fluorescence ;
un moyen pour moduler les intensités relatives des première et seconde lumières d'excitation l'une par rapport à l'autre avant l'acquisition des première et deuxième images de fluorescence ; et
un moyen pour traiter (18) les première et deuxième images de fluorescence pour extraire une troisième image de fluorescence représentant le fluorophore cible, dans lequel le moyen pour moduler les intensités relatives comprend :
un moyen pour identifier une région de longueurs d'onde dans les première et seconde émissions de fluorescence, dans lequel la région de longueurs d'onde est une région où une émission résultant du fluorophore est présente dans la première émission de fluorescence et absente dans la seconde émission de fluorescence ;
un moyen pour sélectionner une bande d'ondes hors de la région de longueurs d'onde ;
un moyen pour calculer dans la bande d'ondes sélectionnée un rapport des intensités relatives des première et seconde émissions de fluorescence l'une par rapport à l'autre ; et
un moyen pour ajuster les intensités relatives des première et seconde excitations l'une par rapport à l'autre pour ajuster la première émission de fluorescence correspondante, la seconde émission de fluorescence ou les deux jusqu'à ce qu'un rapport calculé approprié soit obtenu.

14. Système selon la revendication 13, dans lequel la bande d'ondes hors de la région de longueurs d'onde comprend une ou plusieurs longueurs d'onde dans des spectres de fluorescence résultant des première et seconde émissions de fluorescence.

15. Système selon la revendication 13 ou 14, dans lequel le moyen pour calculer le rapport des intensités relatives des première et seconde émissions de fluorescence comprend un moyen pour diviser une valeur d'aire sous la courbe correspondant à la première émission de fluorescence par une valeur d'aire sous la courbe correspondant à la seconde émission de fluorescence.

16. Système selon l'une quelconque des revendications 13 à 15, dans lequel la première lumière d'excitation présente une longueur d'onde d'environ 405 nm et la seconde lumière d'excitation présente une longueur d'onde d'environ 450 nm quand le fluorophore cible est la porphyrine.

17. Système selon l'une quelconque des revendications 13 à 16, dans lequel la bande d'ondes sélectionnée est d'environ 600 nm et dans lequel le rapport calculé est d'environ 1.

18. Système selon l'une quelconque des revendications 13 à 17, dans lequel le moyen pour traiter les première et deuxième images de fluorescence pour extraire la troisième image de fluorescence représentant le fluorophore cible comprend un moyen pour soustraire la deuxième image de fluorescence de la première image de fluorescence.

19. Système selon l'une quelconque des revendications 13 à 18, dans lequel le moyen d'éclairage comprend un module d'éclairage comprenant une source d'excitation de fluorescence, la source d'excitation de fluorescence étant configurée de manière fonctionnelle pour générer les première et seconde lumières d'excitation.

20. Système selon la revendication 19, dans lequel le module d'éclairage comprend en outre un élément optique configuré de manière fonctionnelle pour mettre en forme et guider les première et seconde lumières d'excitation sortant du module d'éclairage.

21. Système selon la revendication 20, dans lequel l'élément optique comprend une lentille, un guide de lumière, un diffuseur, ou une combinaison de ceux-ci.

22. Système selon l'une quelconque des revendications 13 à 21, dans lequel le moyen pour acquérir comprend un module d'acquisition d'émission de fluorescence, le module d'acquisition d'émission de fluorescence comprenant un capteur d'image.

23. Système selon la revendication 22, dans lequel le module d'acquisition d'émission de fluorescence comprend en outre un élément optique disposé devant le capteur d'image configuré de manière fonctionnelle pour capturer, filtrer, et diriger les première et seconde émissions de fluorescence.

24. Système selon l'une quelconque des revendications 13 à 23, dans lequel le moyen pour traiter comprend un module de processeur.

25. Système selon la revendication 24, dans lequel le module de processeur est configuré de manière fonctionnelle pour commander une opération du moyen pour éclairer, pour commander une opération du moyen pour acquérir, ou une combinaison de ceux-ci.

26. Système selon l'une quelconque des revendications 13 à 25, dans lequel la matière biologique est prétraitée par photoblanchiment.

27. Système selon l'une quelconque des revendications 13 à 26, dans lequel le fluorophore cible est endogène, exogène, ou une combinaison de ceux-ci.

28. Système selon la revendication 27, dans lequel le fluorophore endogène est la porphyrine, un précurseur de porphyrine, un analogue de porphyrine, un dérivé de porphyrine, un conjugué de porphyrine, un liposome de porphyrine, une nanovésicule de porphyrine, ou une combinaison de ceux-ci.

29. Système selon la revendication 28, dans lequel la porphyrine comprend une coproporphyrine, une uroporphyrine, une protoporphyrine, ou une combinaison de celles-ci.

30. Système selon la revendication 27, dans lequel le fluorophore exogène est un colorant fluorescent, un agent induisant une fluorescence, ou une combinaison de ceux-ci.

31. Méthode selon l'une quelconque des revendications 1 à 11, utilisation selon la revendication 12, ou système selon l'une quelconque des revendications 13 à 30, dans lequel la matière biologique comprend un tissu biologique, un fluide biologique, ou une fraction de ceux-ci.

32. Méthode selon l'une quelconque des revendications 1 à 11, utilisation selon la revendication 12, ou système selon l'une quelconque des revendications 13 à 30, dans lequel la matière biologique comprend un organe, une cellule, une lignée cellulaire, un constituant cellulaire dérivé d'un ou localisé dans un mammifère.

33. Méthode selon l'une quelconque des revendications 1 à 11, utilisation selon la revendication 12, ou système selon l'une quelconque des revendications 13 à 30, dans lequel la matière biologique comprend un tissu sain, malade ou cancéreux.

34. Méthode selon l'une quelconque des revendications 1 à 11, utilisation selon la revendication 12, ou système selon l'une quelconque des revendications 13 à 30, dans lequel la matière biologique comprend une section de tissu destinée à être utilisée en histochimie, immunohistochimie, cytochimie, immunofluorescence, immunobuvardage, ou dans une application d'imagerie associée à la fluorescence.
